Europäisches Patentamt

European Patent Office

Office européen des brevets

(19)

(11) Veröffentlichungsnummer : **0 166 011**
**B1**

# (12) EUROPÄISCHE PATENTSCHRIFT

(45) Veröffentlichungstag der Patentschrift :
26.04.89

(21) Anmeldenummer : 84107260.6

(22) Anmeldetag : 22.06.84

(51) Int. Cl.⁴ : **G 09 B 5/00, A 61 B 5/08**

(54) **Steuergerät zum einschalten einer Lehrvorrichtung, welche optische und/oder akustische Lerninformationen übermittelt.**

(43) Veröffentlichungstag der Anmeldung :
02.01.86 Patentblatt 86/01

(45) Bekanntmachung des Hinweises auf die Patenterteilung : 26.04.89 Patentblatt 89/17

(84) Benannte Vertragsstaaten :
AT BE CH DE FR GB IT LI LU NL SE

(56) Entgegenhaltungen :
DE—A— 2 544 383
DE—A— 3 028 120
DE—A— 3 109 026
DE—B— 2 521 412

(73) Patentinhaber : Gelsen, Karl-Heinz
Amselstieg 36
D-2080 Pinneberg (DE)

(72) Erfinder : Jansen, Hansjürgen
Schillerstrasse 11b
D-2080 Pinneberg (DE)
Erfinder : Gelsen, Karl-Heinz
Amselstieg 36
D-2080 Pinneberg (DE)

(74) Vertreter : Säger, Manfred, Dipl.-Ing. et al
Lesser, Flügel & Säger Patentanwälte Richard-Strauss-Strasse 56 Postfach 81 05 40
D-8000 München 80 (DE)

## Beschreibung

Die Erfindung betrifft ein Steuergerät gemäß dem Oberbegriff des Hauptanspruchs.

Biofeedback-Geräte, auch solche, die die Atemtätigkeit erfassen und an den Probanden durch akustische und/oder optische Signale rückmelden, sind an sich bekannt (DE-A-31 09 026).

Es ist ferner ein Steuergerät gemäß dem Oberbegriff des Hauptanspruchs bekannt (DE-B 25 21 412). Dieses dient zur Erstellung eines Lernprogrammes, wobei Lerninhalte und elektrische Reize sowie Muskelsignale gleichzeitig auf einen Probanden einwirken. Außerdem wird die Lerninformation nach Intensität und Geschwindigkeit gesteuert zugeführt. Dieses Programm wird anhand eines Kontrollstudenten und dessen Reaktionen erstellt. Viele den Kontrollstudenten ähnliche Studenten, was mit einer Auswahl geschieht, können mit diesem Lernprogramm optimal arbeiten. Soweit das Lernprogramm nicht auf den Kontrollstudenten, sondern nur einem diesem ähnlichen Studenten angewendet wird, findet offenbar keine Rückkoppelung der von diesem Studenten gezeigten physischen und psychischen Signale statt ; vielmehr wird er entsprechend der gespeicherten Reaktionen des Kontrollstudenten von den Speichereinrichtungen mittels Muskelreizen oder sonstigen elektrischen Reizen beaufschlagt.

Soweit der Kontrollstudent betroffen ist, so werden zwar die Verfassung des Studenten automatisch berücksichtigt und dieser bei bedarf suggestiven Signalen und Reizimpulsen zur Erhöhung seiner Aufnahmefähigkeit ausgesetzt. So kann mittels einer Verzögerungsschaltung die Wiedergabe der Lerninformationen verzögert sowie bis zur Wahrnehmungsschwelle abgeschwächt werden.

Von Nachteil bei dieser bekannten gattungsgemäßen Vorrichtung ist die Tatsache, daß einerseits die Lerninformationen bei anderen als Kontrollstudenten nur entsprechend einem aufgezeichneten Speicherinhalt gesteuert, also nicht Biofeedback-mäßig rückgekoppelt geregelt zugegeben wird und außerdem die Lerninformation, wenngleich verzögert, so doch stets wiedergegeben wird, auch wenn sich der Kontrollstudent in einem nicht aufnahmefähigen Zustand befindet.

Der Erfindung liegt die Aufgabe zugrunde, eine gattungsgemäße Vorrichtung gemäß dem Oberbegriff des Hauptanspruchs so weiterzubilden, daß damit alle Probanden individuell gesteuert mit Lerninformation versorgt werden können und außerdem in Zeiten schlechter Aufnahmefähigkeit die Wiedergabe der Lerninformation unterbrochen wird.

Diese Aufgabe wird bei einem gattungsgemäßen Steuergerät gemäß dem Oberbegriff des Hauptanspruchs erfindungsgemäß durch dessen kennzeichnende Merkmale gelöst.

Das Steuergerät gemäß der Erfindung macht hierbei von der Erkenntnis gebrauch, daß im alpha-Zustand, d. h. einem Zustand, in welchem z. B. die Atemfrequenz auf durchschnittlich sieben Atemzüge pro Minute abfällt, eine bessere Lern- und Merkfähigkeit erzielt werden kann. Um den Probanden zu erleichtern, in den alpha-Zustand zu kommen, wird das Analogsignal der Atemfrequenz ihm akustisch und/oder optisch angezeigt, wodurch eine Biofeedback-Wirkung erzielt wird. Durch diese kann der Proband leichter und sicherer in den alpha-Zustand gelangen. Mit ganz besonderem Vorteil ist es auch möglich, die akustische und/oder optische Anzeige des Analogsignals im eingeschalteten Zustand der Lehrvorrichtung, während diese also Lerninformationen übermittelt, parallel zu dieser geschaltet zu lassen.

Zweckmäßige Ausgestaltungen und Weiterbildungen der Erfindung sind in den Unteransprüchen gekennzeichnet.

Ein Ausführungsbeispiel der Erfindung wird nachfolgend unter Bezugnahme auf die Zeichnung näher erläutert. Es zeigt :

Fig. 1 ein schematisches Blockdiagramm für das Steuergerät gemäß der Erfindung und

Fig. 2 den Aufbau eines Blocks gemäß Fig. 1.

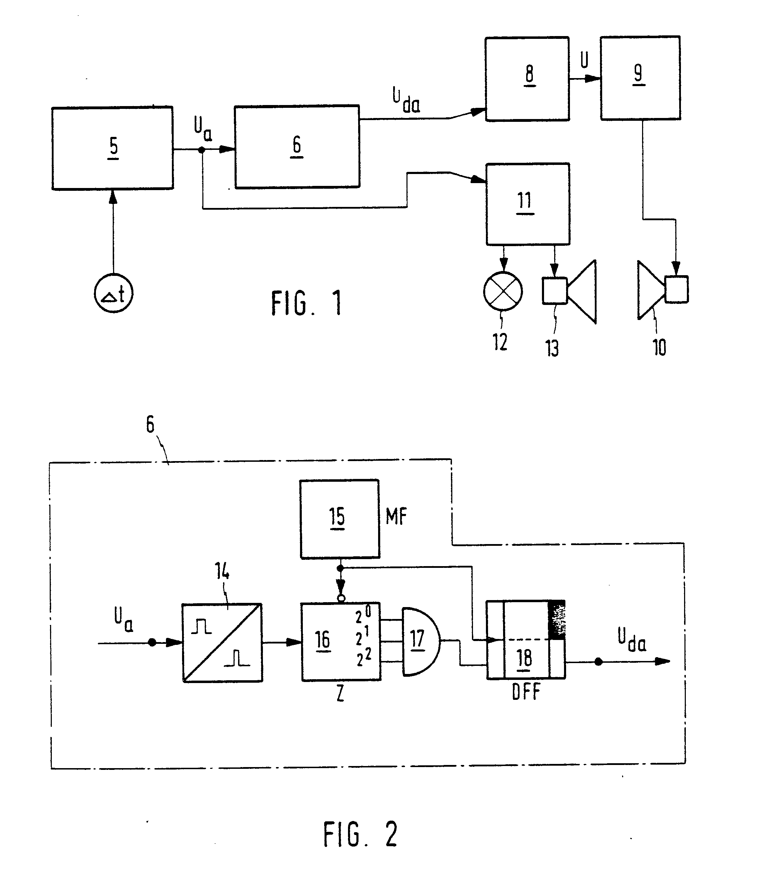

In Fig. 1 ist mit 5 ein Wandler bezeichnet, der die Atemfrequenz eines Probanden in ein dieser entsprechenden, elektrisches Ausgangs-Analog-signal $U_a$ umwandelt. Der Wandler mißt die Temperatur des Atems und wird beim Ausatmen von der verbrauchten körperwarmen Luft umspült ; beim Einatmen hingegen von raumtemperierter Luft. Die Analog-Spannung $U_a$ schwankt im Rhythmus und der Stärke der Atmung und wird einem Detektor 6 zugeführt, welcher feststellt, ob der Alpha-Zustand erreicht ist. In diesem Falle gibt er ein digitales Ausgangssignal $U_{da}$ an eine Schalteinheit 8, die ihrerseits die eigentliche Lehrvorrichtung 9 zur optischen und/oder akustischen Übermittlung von Lerninformation, beispielsweise über einen Lautsprecher 10 einschaltet.

Zugleich kann das Analog-Signal einem Signalgenerator 11 zugeführt werden, der in seinem Rhythmus eine Lampe 12 und/oder einen Lautsprecher 13 ansteuert.

Fig. 2 zeigt den schematischen Aufbau des Detektors 6 gemäß Fig. 1. Das Analog-Signal $U_a$ wird einem Schmitt-Trigger 14 zugeführt, der aus dem Analog-Signal Digitalimpulse macht. Diese werden einem Dualzähler 16 zugeführt. Die Ausgänge des Zählers sind über ein UND-Gatter 17 miteinander verbunden. Der Ausgang des UND-Gatters 17 ist an den D-Eingang des D-Flipflop 18 angeschlossen, dessen Ausgangssignal dem Digitalsignal $U_{da}$ entspricht.

Ferner ist ein Monoflop 15 vorgesehen, dessen Ausgang mit dem asynchronen Rücksetzeingang des Zählers 16 und dem Takteingang des D-Flipflops 18 verbunden ist. Das Monoflop 15 gibt Periodisch jede Minute einen Impuls ab. Mit diesem Impuls wird der am D-Eingang des D-Flopflops 18 liegende Wert in das Flipflop übernommen und zugleich der Zähler 16 für einen neuen Zählzyklus zurückgestellt. Zählt dieser im Laufe einer Minute sieben Impulse aufgrund der Atemfrequenz des im Alpha-Zustand befindlichen Probanden, so führt jeder seiner Ausgänge ($2^0$, $2^1$ und $2^2$) eine logische Eins. Nur in diesem Falle gibt das UND-Gatter ein Ausgangssignal in Form einer logischen Eins ab, so daß das Ausgangssignal des D-Flipflops dem Digitalsignal entspricht und immer dann eine logische Eins führt, wenn der Alpha-Zustand erreicht ist.

**Patentansprüche**

1. Steuergerät für eine Lehrvorrichtung (9), welche an einen Probanden optische und/oder akustische Lerninformationen übermittelt, mit einem den Hautwiderstand oder einem den Puls oder Atem nach Intensität und Rhythmus in ein elektrisches Analogsignal konvertierenden Wandler (5) mit einem dem Wandler nachgeordneten Detektor (6) zur Abgabe eines elektrischen Digitalsignals bei Erreichen eines bestimmten Wachschlafzustandes und mit einer dem Detektor (6) nachgeordneten Schalteinheit, dadurch gekennzeichnet, daß der Detektor (6) bei Erreichen des Alpha-Zustandes als Wachschlafzustand das Digitalsignal abgibt, daß die Schalteinheit zum Einschalten der Lehrvorrichtung (9) bei Anlage des Digitalsignals dient und daß das Analogsignal (Ua) an einem Signalgenerator (11) anliegt, der ein Ausgangssignal entsprechend dem Analogsignal einer Lampe (12) und/oder einem Lautsprecher (13) zuführt, um den Probanden das Analogsignal (Ua) anzuzeigen, wodurch eine Biofeedback-Wirkung erzielt wird, durch die der Proband leichter in den Alpha-Zustand gelangen kann.

2. Gerät nach Anspruch 1, dadurch gekennzeichnet, daß der Detektor (6) aufweist : einen das Analogsignal ($U_a$ in Digitalimpulse umwandelnden Schmitt-Trigger (14), einen Zähler (16) für die Digitalimpulse, ein Gatter (17) für die Ausgänge des Zählers (16) zur Feststellung des Zählerinhaltes, ein D-Flip-Flop (18), dessen D-Eingang mit dem Ausgang des Gatters (17) verbunden ist und dessen Ausgang das Digital-Signal ($U_{da}$) führt und ein periodisch angestoßenes Monoflop, dessen Ausgang sowohl mit den Rücksetzeingang des Zählers (16) als auch dem Takteingang des D-Flip-Flops (18) verbunden ist.

3. Gerät nach Anspruch 2, dadurch gekennzeichnet, daß der Zähler (18) ein Dualzähler ist, daß das Gatter (17) ein UND-Gatter ist, und daß das Monoflop (15) eine Impulswiederholungsfrequenz von einer Minute aufweist.

**Claims**

1. Control apparatus for a teaching device (9) which transmits to a test student optical and/or acoustic learning information, having a transducer (5) converting the skin resistance or the pulse or breath according to intensity and rhythm into an electrical analogue signal, a detector (6), connected to the transducer, for emitting an electrical digital signal when a specific state of being awake or being asleep is reached, and a switching unit connected to the detector (6), characterised in that the detector (6) emits the digital signal when the alpha-state as a state of being awake or being asleep is reached, in that the switching unit serves to switch on the teaching device (9) when the digital signal is applied, and in that the analogue signal ($U_a$) is applied to a signal generator (11), which, according to the analogue signal, feeds an output signal to a lamp (12) and/or a loudspeaker (13) in order to indicate the analogue signal ($U_a$) to the test student, thus achieving a bio-feedback effect which enables the test student to reach the alpha-state more easily.

2. Apparatus according to claim 1, characterised in that the detector (6) has a Schmitt-trigger (14) which converts the analogue signal ($U_a$) into digital pulses, a counter (16) for the digital pulses, a gate (17) for the outputs of the counter (16) for determining the counter data, a D-flip-flop (18), whose D-input is connected to the output of the gate (17) and whose output carries the digital signal ($U_{da}$), and a monoflop which receives periodic pulses and whose output is connected both to the reset input of the counter 16 and to the clock input of the D-flip-flop (18).

3. Apparatus according to claim 2, characterised in that the counter (18) is a binary counter, that the gate (17) is an AND-gate, and that the monoflop (15) has a pulse repetition frequency of 1 minute.

**Revendications**

1. Appareil de commande pour un dispositif d'apprentissage (9) qui retransmet à un patient des informations optiques et/ou acoustiques d'apprentissage, cet appareil comportant un convertisseur (5) transformant en un signal électrique analogique la résistance de la peau ou bien un convertisseur (5) transformant en un signal électrique analogique le pouls ou la respiration en intensité et en rythme, et avec un détecteur (6) disposé à la suite du convertisseur pour délivrer un signal électrique numérique lorsqu'un état déterminé de veille-sommeil est atteint, ainsi qu'avec une unité de commutation disposée à la suite du détecteur (6), appareil de commande caractérisé en ce que le détecteur (6) délivre le signal numérique lorsque l'état alpha constituant l'état de veille-sommeil est atteint, l'unité de commutation ayant pour fonction, lorsque le signal numérique lui est appliqué, de mettre en circuit le dispositif d'apprentissage (9), et le signal analogique ($U_a$) étant appliqué à un générateur de signaux (11) qui fournit à une lampe (12) et/ou à un haut-parleur (13) un signal de sortie correspondant au signal analogique, pour indiquer au patient le signal analogique ($U_a$), grâce à quoi un effet biologique de rétro-action (biofeedback) est obtenu, grâce auquel le patient peut plus facilement parvenir à l'état alpha.

2. Appareil selon la revendication 1, caractérisé en ce que le détecteur (6) comporte :

- une bascule de Schmitt (14) convertissant le signal analogique ($U_a$) en impulsions numériques,
- un compteur (16) pour les impulsions numériques,
- une porte (17) pour les sorties du compteur (16) afin de déterminer le contenu de ce compteur,
- une bascule bistable (18) dont l'entrée D est reliée à la sortie de la porte (17), et dont la sortie achemine le signal numérique ($U_{da}$),
- une bascule monostable, déclenchée périodiquement, dont la sortie est reliée aussi bien à l'entrée de remise à l'état initial du compteur (16) qu'à l'entrée de synchronisation de la bascule bistable (18).

3. Appareil selon la revendication 2, caractérisé en ce que le compteur (18) est un compteur de nombre de base 2, que la porte (17) est une porte ET, et que la bascule monostable (15) a une fréquence de répétition d'impulsions de 1 minute.

## FIG. 1

## FIG. 2